# EUROPEAN PATENT APPLICATION

(11) **EP 3 922 312 A1**
(43) Date of publication of application: **15.12.2021**
(21) Application number: 20305633.8
(22) Date of filing: 10.06.2020
(51) Int. Cl.: A61P 31/14, A61K 9/08, A61K 47/20, A61K 31/05

(54) **COMPOUND AND METHOD FOR THE TREATMENT OF CORONAVIRUSES**

(71) Applicant: Apteeus, 59000 Lille (FR)
(72) Inventor: The designation of the inventor has not yet been filed
(74) Representative: Cabinet Beau de Loménie

(57) **Abstract**

The invention relates to the use of clofoctol in the prevention or treatment of a disease caused by a coronavirus, in particular Covid-19.

## Description

### Field of the invention

The present invention relates to the use of clofoctol for the treatment of a disease caused by a coronavirus, in particular Covid-19.

### Background of the invention

Coronaviruses are belonging to the family *Coronaviridae* (order *Nidovirales*) and include viruses with a single-strand, positive-sense RNA genome approximately 26-32 kilobases in size. The *Coronaviridae* family includes Alpha-coronavirus (alphaCoV), Beta-coronavirus (betaCoV), Delta-coronavirus (deltaCoV) and Gamma-coronavirus (gammaCoV). Bats and rodents are thought to be the reservoir for alphaCoV and betaCoV. Currently, it is less clear which animals serve as the reservoir for deltaCoV and gammaCoV. Coronaviruses are named according to their appearance under the electron microscope, the viruses look like they are covered with pointed structures that surround them like a corona or crown due to the presence of spike glycoproteins on their envelope.

Three coronaviruses have crossed the species barrier to cause deadly pneumonia in humans since the beginning of the 21^{st} century: Severe Acute Respiratory Syndrome coronavirus (SARS-CoV), Middle East Respiratory Syndrome coronavirus (MERS-CoV), and SARS-CoV2 (also known as 2019-nCoV). SARS-CoV2 is an enveloped, positive-sense, single-stranded RNA virus which belongs to the betaCoV genus, which genus also includes SARS-CoV and MERS-CoV. SARS-CoV2 shares 89% nucleotide identity with bat SARS-like CoV and 82% identity with human SARS-CoV.

Coronavirus entry into host cells is mediated by the transmembrane spike (S) glycoprotein that forms homotrimers protruding from the viral surface. It has been shown that in addition to direct membrane fusion, SARS-CoV could enter cells through an endocytic route, and this endocytic infection lead to viral gene expression (Cell Research 2008; 18: 290-301). In respect of SARS-CoV2, it has been suggested that ACE2 (angiotensin-converting enzyme 2) mediates SARS-CoV2 S-mediated entry into cells, establishing it as a functional receptor for this newly emerged coronavirus; SARS-CoV2 S engages human ACE2 with comparable affinity to SARS-CoV S (Cell 2020; 180: 281-292). It has also been shown that SARS-CoV-2 uses transmembrane protease, serine 2 (TMPRSS2) for S protein priming (Cell 2020; 181: 271-280).

Since the emergence of SARS-CoV2, and the outbreak of the associated disease, Covid-19, a number of drugs, either investigational or already on the market, have been tested for their potential efficacy in treating / alleviating the effects of Covid-19. To the knowledge of the inventors, none of these drugs have been shown to block the virus cycle when both entry pathways of SARS-CoV2 are available in the cells, i.e. the endocytic entry and the fusion entry.

Clofoctol (2-(2,4-Dichlorobenzyl)-4-(1,1,3,3 -tetramethylbutyl)phenol, CAS number 37693-01-9) is a bacteriostatic antibiotic, indicated for the treatment of infections caused by Gram-positive bacteria. It was first described in French patent application 2 101 076. This compound has subsequently been shown to activate the unfolded protein response pathways, making it a potential drug candidate for the treatment of prostate cancer (British Journal of Pharmacology 2014; 171: 4478-4489). More recently, clofoctol has been show to activate EBV lytic gene expression (Journal of Virology 2019; 93(20): e00998-19) and to suppress glioma stem cell proliferation by activating KLF13 (J Clin Invest. 2019; 129(8):3072-3085).

### Summary of the invention

It has now been found that clofoctol blocks the virus cycle when both entry pathways of SARS-CoV2 are available in the cells, i.e. the endocytic entry and the fusion entry. As a consequence, clofoctol has been found to be effective in suppressing the cytopathic effect induced by SARS-CoV2, and in reducing the viral load in cells infected with the virus. Accordingly, the invention relates to clofoctol for use in the prevention or treatment of a disease caused by a coronavirus such as SARS-CoV, MERS-CoV or SARS-CoV2. The invention also relates to a method of treating a disease caused by a coronavirus, such as SARS-CoV, MERS-CoV or SARS-CoV2, which comprises administering clofoctol to a subject in need thereof.

### Brief description of the figures

Figure 1 and Figure 3 show the efficacy of various concentrations of clofoctol on the cytopathic effect of SARS-CoV2 infecting Vero81 cell lines.
Figure 2 and Figure 4 show the efficacy of various concentrations of clofoctol on growth inhibition of Vero81 cell lines, transduced with the TMPRSS2 gene, and infected with SARS-CoV2.
Figure 5 shows the effect of clofoctol on the viral load of infected Vero81 cells and infected Vero81 cells transduced with TMPRSS2.

### Detailed description of the invention

In the context of the present disclosure, the various embodiments described hereafter can be combined.

In a first aspect, the present invention relates to clofoctol for use in the prevention or treatment of a disease caused by a coronavirus such as SARS-CoV, MERS-CoV or SARS-CoV2.

In one embodiment, the coronavirus is SARS-CoV2, and the corresponding disease is Covid-19.

In one embodiment, clofoctol is administered in combination with at least one other active principle (small molecule or biological) which is or has been under investigation for the treatment of SARS-CoV, MERS-CoV or SARS-CoV2. The administration of the at least one other active principle can be simultaneous, sequential or over a period of time, with respect to the administration of clofoctol.

In one embodiment the at least one other active principle is selected from:
Abemaciclib, almitrine bismesylate, amodiaquine, anakinra, angiotensin 1-7, anidulafungin, apixaban, aspirin, avatrombopag, azithromycin, baricitinib, bazedoxifene, berbamine, brexpiprazole, bromhexine, camostat, canakinumab, cepharanthine, ceritinib, cetylpyridinium, chloroquine, chlorpromazine, ciclesonide, clomifene citrate, cobicistat, croconazole, cyclosporine, danoprevir, darunavir, digitoxin, digoxin, dihydroartemisinine, dihydrogambogic acid, diltiazem, dronedarone, drotaverine, ebastine, eltrombopag, emapalumab, ethaverine, favipiravir, fingolimod, flunarizine, gilteritinib, harringtonine, hematoporphyrin, hexachlorophene, hydroxychloroquine, hydroxyprogesterone caproate, IMU-838, interferon Beta-1A, interferon Beta-1B, isoosajin, isopomiferin, isotretinoin, ivacaftor, ivermectin, JS016, ketoconazole, lidoflazine, loperamide, lopinavir, loratadine, loteprednol etabonate, lusutrombopag, LY-CoV555, mefloquine, nafamostat, niclosamide, nitazoxanide, omacetaxine mepesuccinate, osajin, oseltamivir osimertinib, otilimab, ouabain, oxiconazole, oxyclozanide, ozanimod, papaverine, pegylated interferon lambda, perhexiline maleate, pexidartinib, phenazopyridine, polydocanol, posaconazole, recombinant human interferon α1β, recombinant Interferon Alfa-2b, regorafenib, remdesivir, ribavirin, ritonavir, ruxolitinib, sitagliptin, sofosbuvir, sonidegib, sorafenib, tamoxifen, thalidomide, thimerosal, thioridazine, thymosin alpha 1, tilorone, tioguanine, tocilizumab, toremifene, triparanol, tyrphostin, umifenovir.

In one embodiment, clofoctol is administered in the form of a pharmaceutical composition comprising at least one pharmaceutically acceptable excipient (in addition to clofoctol). Excipients, pharmaceutical compositions and method for their preparation are well known in the art (see e.g. Handbook of pharmaceutical Excipients, Rowe et al, seventh edition, June 2012; Rules and Guidance For Pharma Manufacturers and distributors 205, Medecines and Healthcare products Regulatory Agency, London UK).

In another aspect, the present invention relates to a method of preventing or treating a disease caused by a coronavirus, such as SARS-CoV, MERS-CoV or SARS-CoV2, which comprises administering clofoctol to a subject in need thereof.

In one embodiment, the coronavirus is SARS-CoV2, and the corresponding disease is Covid-19.

In one embodiment the subject has one or more of the following conditions or diseases: overweight, obesity, diabetes, hyperlipidaemia, arterial hypertension, cardiovascular disease, chronic kidney disease, immunosuppression.

In one embodiment, the method further comprises the administration to the subject of at least one other active principle. The administration of the at least one other active principle can be simultaneous, sequential or over a period of time, with respect to the administration of clofoctol.

In one embodiment the at least one other active principle is as defined above.

Clofoctol has been shown to suppress the cytopathic effect of hSARS-CoV2 in Vero81 cells as well as in Vero81 cells transduced with TMPRSS2. Vero81 are permissive to the virus with an entry way based on endocytosis. When transduced with TMPRSS2, Vero81 cell becomes permissive to the virus with 2 entry ways, endocytosis and fusion.

Clofoctol has also been shown to reduce the viral load in Vero81 cells and in Vero81 cells transduced with TMPRSS2.

The following examples have been included for the purpose of illustration, and are not to be construed as limiting in any manner.

### Examples

### Virus isolation & amplification

Human coronavirus SARS-CoV2 (hSARS-CoV2) was isolated from patient samples and annotated BetaCoV/France/IDF0372/2020. The virus was then amplified in VERO-81cells expressing TMPRSS2, cultivated on plates and incubated until the complete destruction of cell monolayers. After harvesting, virus titrations were performed by serial 10-fold dilutions of supernatants (10⁻¹ to 10⁻⁸) distributed onto VERO-81 cells incubated with DMEM supplemented with 2% FBS (Fetal Bovine Serum). The plates were incubated for 5 days at 37 °C with 5% CO₂. Afterwards, the plates were examined using an inverted microscope (ZEISS Primovert) to evaluate the extent of the virus-induced cytopathic effect in cell culture. Calculation of estimated virus concentration was carried out by the Spearman and Karber method and expressed as log10 TCID50/mL (50% tissue culture infectious dose). The limit of detection of the method was 1.5 TCID50/ml.

### TMPRSS2 transduction

Vero-81 cells were transduced with a lentivirus expressing TMPRSS2. TMPRSS2 was cloned into pTRIP vector, lentivectors were produced by transfection of HEK293T cells with pTRIP-TMPRSS2, phCMV-VSVG and HIV gag-pol with Turbofect^{™} (ThermoFischer) according to the manufacturer's instruction. Supernatants containing lentiviral vectors were used to transduce cells. Then cells were used in experiments 72h after the transduction.

### Measure of the cytopathic effect

Cell culture: Vero cells [ATCC^{®} CCL-81^{™}, ECACC, Sigma-Aldrich, France, hereafter "Vero81" cells] were cultured in Dulbecco's modified Eagle medium (DMEM) supplemented with 10% fetal bovine serum (FBS) and 1% L-glutamine in a humidified atmosphere of 5% CO₂ at 37 °C.

Tested compound: solutions of the clofoctol were prepared in water or DMSO at concentrations required to achieve the desired concentrations in the experiment. Solutions were dispensed onto the living cells. The maximum concentration of DMSO was 0.15%. Cell infection: cells were infected after addition of clofoctol at various concentrations or control solvents. The multiple of infection was 0.01.

Reagents: Hoechst 33342, Trihydrochloride, trihydrate, (Ref H3570, ThermoFischer) and Propidium Iodide (Ref P1304MP, ThermoFisher) were respectively used at 10 µg/ml and 1 µg/ml respectively to monitor the viral cytopathic effect (CPE) of clofoctol. They were both added after an incubation time of 72h.

Results were obtained by analyzing the percentage of PI-positive nuclei, surrogate of the toxicity of the virus exerts on cells, and number of nuclei corresponding to the cell growth inhibition exerted by the virus.

### Measure of the viral load

Cell culture: Vero81 cells and Vero-81 transduced with TMPRSS2 were cultured in Dulbecco's modified Eagle medium (DMEM) supplemented with 10% fetal bovine serum (FBS) and 1% L-glutamine in a humidified atmosphere of 5% CO₂ at 37 °C.

Tested compound: solutions of clofoctol were prepared in water or DMSO at concentrations required to achieve the desired concentrations in the experiment. Solutions were dispensed onto the living cells. The maximum concentration of DMSO was 0.15%. Cell infection: cells were infected after addition of clofoctol at various concentrations or control solvents. The multiple of infection was 0.25.

Results were obtained by dosing viral RNA related to total cellular RNA, surrogate of the viral loads.

RNA dosing: after 24 hours of incubation, total cellular RNA was extracted by using the Nucleospin RNA kit (Macherey-Nagel) as recommended by the manufacturer and SARS-CoV-2 genome were measured by quantitative RT-PCR. RNA was subjected to reverse transcription by using the high-capacity cDNA reverse transcription kit (Applied Biosystems). Then real time PCR was performed with TaqMan universal PCR master mix (Applied Biosystems) in a QuantStudio 3. Primers and probe (WHO protocol) targeting the open reading frame for the E gene were used. Standard curves were created by using in vitro transcribed RNA corresponding to the amplicon.

### Example 1

The effect of clofoctol on Vero81 cells infected with hSARS-CoV2 was assessed by adding a range of concentrations of clofoctol, following 1:3 dilutions, prior to infection with hSARS-CoV2 at MOI 0.01 (Multiple Of Infection (MOI) 0.01 means that the amount of virus used is 0.01 per 1 cell) for 72 hours. Biological duplicates with two technical replicates were performed.

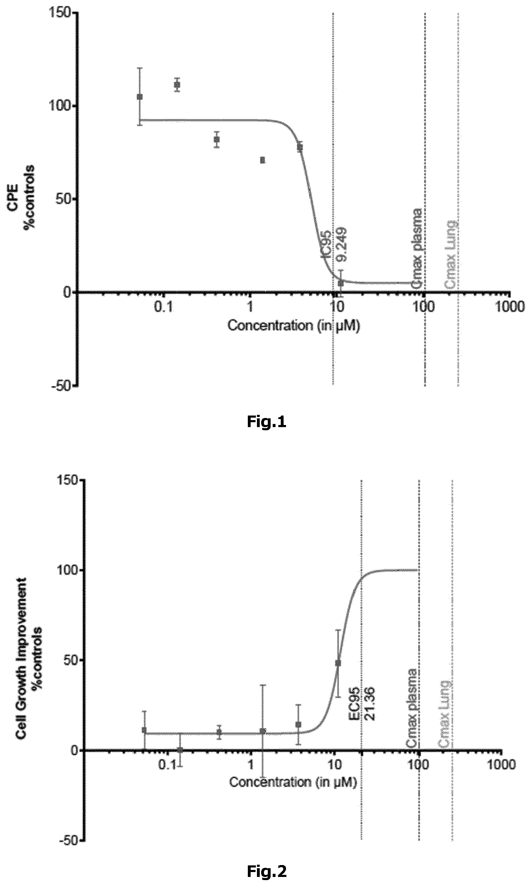

The results were obtained by analyzing the percentage of PI-positive nuclei expressed as the percentage of CPE relative to a scale defined by positive (0% infected cells) and negative (100% infected cells) controls. It can be seen from Figure 1 that clofoctol has a concentration reducing 95% (IC₉₅) of the CPE of 9.2µM.

### Example 2

The effect of clofoctol on Vero81 cells infected with hSARS-CoV2 was assessed by adding a range of concentrations of clofoctol, following 1:3 dilutions, prior to infection with hSARS-CoV2 at MOI 0.01 for 72 hours. Biological duplicates or triplicates with three technical replicates were performed.

The results were obtained by analyzing the number of living cells expressed as the percentage of cell growth improvement relative to a scale defined by normal cell growth (not infected controls) and no cell growth (infected controls). It can be seen from Figure 2 that the concentration of clofoctol at which 95% of its maximum response is observed (EC₉₅) is 21µM.

### Example 3

The effect of clofoctol on TMPRSS2-transduced Vero81 cells infected with hSARS-CoV2 was assessed by adding a range of concentrations of clofoctol, following 1:3 dilutions, prior to infection with hSARS-CoV2 at MOI 0.01 for 72 hours. Biological triplicates were performed.

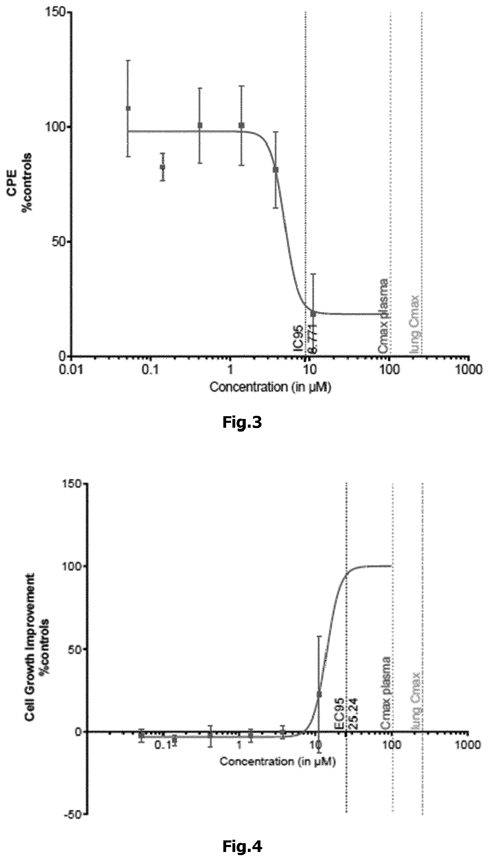

The results were obtained by analyzing the percentage of PI-positive nuclei expressed as the percentage of CPE relative to a scale defined by positive (0% infected cells) and negative (100% infected cells) controls. It can be seen from Figure 3 that clofoctol has an IC₉₅ of 8.7µM.

### Example 4

The effect of clofoctol on TMPRSS2-transduced Vero81 cells infected with hSARS-CoV2 was assessed by adding a range of concentrations of clofoctol, following 1:3 dilutions, prior to infection with hSARS-CoV2 at MOI 0.01 for 72 hours. Biological triplicates were performed.

The results were obtained by analyzing the number of living cells expressed as the percentage of cell growth improvement relative to a scale defined by normal cell growth (not infected controls) and no cell growth (infected controls). It can be seen from Figure 4 that clofoctol has an EC₉₅ of 25µM.

### Example 5

The effect of clofoctol on Vero81 and TMPRSS2-transduced Vero81 cells infected with hSARS-CoV2 was assessed by adding a range of concentrations of clofoctol, following 1:2,5 dilutions, prior to infection with hSARS-CoV2 at MOI 0.25 for 24 hours. Biological duplicates were performed.

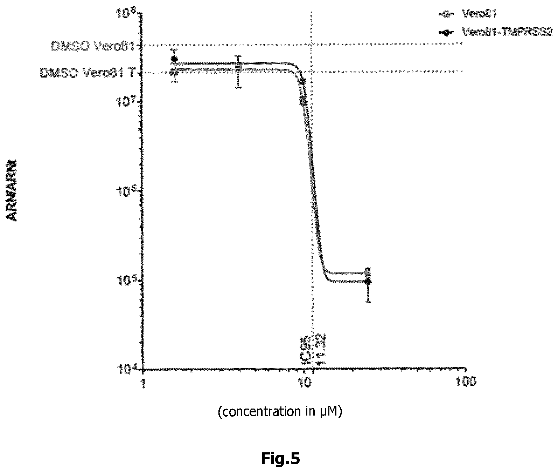

Results were obtained by dosing viral RNA and the viral load was expressed as the ratio of the viral RNA on the total RNA (RNAt) of the sample. It can be seen from Figure 5 that clofoctol has an IC₉₅ of 11µM.

The above results show that the IC₉₅ of clofoctol varies from about 9µM to about 11µM, i.e. values which are about 25 times less than the Cmax (about 250µM) reported for clofoctol in human lung tissue of patients, undergoing pulmonary resection or pneumonectomy, to which clofoctol was administered before or during surgery (Journal of Antimicrobial Chemotherapy 1987; 19: 679-683). Likewise, the EC₉₅ of clofoctol varies from about 21µM to about 25µM, i.e. values which are about 10 times less that the Cmax reported in human lung tissue (see above). These data evidence the value of clofoctol for the prevention or treatment of diseases caused by coronaviruses, such as Covid-19.

## Claims

1. Clofoctol for use in the prevention or treatment of a disease caused by a coronavirus.

2. Clofoctol for the use of claim 1, wherein the coronavirus is SARS-CoV, MERS-CoV or SARS-CoV2.

3. Clofoctol for the use of claim 1, wherein the disease is Covid-19.

4. Clofoctol for the use of any preceding claims, wherein clofoctol is administered in combination with at least one other active principle.

5. Clofoctol for the use of claim 4, wherein the at least one other active principle is administered simultaneously, sequentially or over a period of time.

6. Clofoctol for the use of any preceding claims, wherein clofoctol is administered in the form of a pharmaceutical composition comprising at least one pharmaceutically acceptable excipient.

7. A method of preventing or treating a disease caused by a coronavirus, which comprises administering clofoctol to a subject in need thereof.

8. The method of claim 7, wherein the coronavirus is SARS-CoV, MERS-CoV or SARS-CoV2.

9. The method of claim 7, wherein the disease is Covid-19.

10. The method of any preceding claims, which further comprises administering at least one other active principle to the subject.

11. The method of claim 4, wherein the at least one other active principle is administered simultaneously, sequentially or over a period of time.
